(19) Europäisches Patentamt
European Patent Office
Office européen des brevets

(11) Numéro de publication : 0 347 306 B1

(12) # FASCICULE DE BREVET EUROPEEN

(45) Date de publication du fascicule du brevet : **08.04.92 Bulletin 92/15**

(51) Int. Cl.$^5$ : **A61K 7/00**

(21) Numéro de dépôt : **89401643.5**

(22) Date de dépôt : **13.06.89**

(54) **Composition parfumante, à phase aqueuse continue, ayant une forte concentration en parfum.**

Le dossier contient des informations techniques présentées postérieurement au dépôt de la demande et ne figurant pas dans le présent fascicule.

(30) Priorité : **14.06.88 FR 8807911**

(43) Date de publication de la demande : **20.12.89 Bulletin 89/51**

(45) Mention de la délivrance du brevet : **08.04.92 Bulletin 92/15**

(84) Etats contractants désignés : **AT BE CH DE ES GB GR IT LI LU NL SE**

(56) Documents cités :
**EP-A- 0 043 327**
**FR-A- 2 597 345**
**GB-A- 2 166 107**

(73) Titulaire : **L'OREAL**
**14, Rue Royale**
**F-75008 Paris (FR)**

(72) Inventeur : **Handjani, Rose-Marie**
**17bis, rue Campagne-Première**
**F-75014 Paris (FR)**
Inventeur : **Zabotto, Arlette**
**24, rue Sarrette**
**F-75014 Paris (FR)**
Inventeur : **Ribier, Alain**
**2, boulevard Jourdan**
**F-75014 Paris (FR)**
Inventeur : **Griat, Jacqueline**
**11, Quai de la Baronnie**
**F-94480 Ablon (FR)**

(74) Mandataire : **Michardière, Bernard et al**
**C/O CABINET PEUSCET 68, rue d'Hauteville**
**F-75010 Paris (FR)**

EP 0 347 306 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une composition parfumante, à phase aqueuse continue,ayant une forte concentration en parfum.

Il est connu que les compositions parfumantes peuvent se présenter sous diverses formes : elles se présentent le plus souvent sous forme de solutions hydroalcooliques à fort taux d'alcool. Ces solutions hydroalcooliques ont l'avantage de pouvoir contenir un taux élevé de parfum qui va jusqu'à 25 % en poids ; mais à cause de la présence d'alcool, elles sont irritantes pour la peau et ont tendance à la dessécher.

On connait également des compositions parfumées constituées par des émulsions huile dans l'eau ou eau dans l'huile sous forme de lait ou de crème. Dans ces émulsions, le parfum est dissous dans l'huile et ne peut être introduit qu'en faible quantité.

Dans le brevet FR-A-2 597 345, on a montré que l'on pouvait introduire dans la phase lipidique de vésicules constituée d'une pluralité de couches lipidiques séparées les unes des autres par des couches de phase aqueuse, au moins un lipoprotide mais, dans ces compositions, le parfum n'est éventuellement introduit que comme additif, c'est-à-dire dans une très faible proportion pondérale.

Enfin, on prépare également des parfums peptisés. Mais il y a, dans ce cas, interpénétration de l'agent tensio-actif peptisant dans les particules d'agent parfumant. Dans ce cas l'agent peptisant risque de perturber la note parfumée. De plus, l'agent tensio-actif peptisant risque d'irriter la peau, et il donne un effet collant au produit.

La présente invention concerne une composition parfumante qui ne contient pas d'alcool inférieur et peut cependant renfermer une forte concentration en parfum, dans laquelle la note parfumée ne risque pas d'être dénaturée et qui, loin d'irriter la peau, a au contraire une action adoucissante sur celle-ci par apport de lipides biomimétiques à ceux de la peau.

La présente invention concerne une composition parfumante à phase continue aqueuse ayant une concentration élevée en parfum, caractérisée par le fait qu'elle contient, dans une phase aqueuse continue pouvant éventuellement contenir des adjuvants cosmétiques hydrosolubles :

a) des vésicules, qui sont obtenues à partir d'au moins un lipide nonionique constitué par un dérivé de polyglycérol linéaire ou ramifié,de formule :

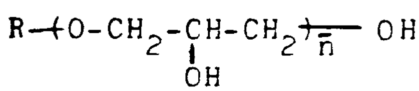

ou

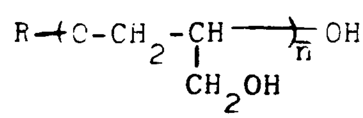

où $\bar{n}$ a une valeur statistique moyenne comprise entre 2 et 6 et où R peut être :

1) ou bien une chaîne aliphatique $R_1$ ou $R_2$ CO, dans laquelle $R_1$ est un radical aliphatique, linéaire ou ramifié, en $C_{12}$-$C_{18}$ et dans laquelle $R_2$ est un radical aliphatique, linéaire ou ramifié, en $C_{11}$-$C_{17}$ ;

2) ou bien un radical

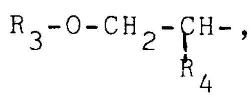

où $R_3$ est un radical $R_1$ ou $R_2CO$ et où $R_4$ est un radical $R_1$, $R_1$ et $R_2$,identiques ou différents, ayant les mêmes significations que ci-dessus indiqué ; ledit ou lesdits lipide(s) non ionique(s) étant associé(s) au cholestérol lorsque R a la signification indiquée sous 1) ci-dessus, ledit (ou lesdits) lipide(s) non-ionique(s) étant associé(s) à un agent stabilisant anionique pris dans le groupe formé par les esters phosphoriques d'alcools gras en $C_{12}$-$C_{22}$, les lipoaminoacides, le sulfate et le phosphate de cholestérol, ledit agent stabilisant anionique étant présent à raison de 1 à 10% en poids par rapport au(x) lipide(s), les vésicules ayant un diamètre moyen compris entre 0,01µm et 1 µm et le(s) lipide(s) non-ionique(s), qui les constitue(nt), représentant de 0,2 à 4% en poids de la composition totale ; et

b) un parfum renfermant au moins une huile essentielle naturelle et/ou un produit synthétique à odeur agréable, essentiellement exempt d'alcool en $C_1$-$C_4$, sous forme de gouttelettes ayant un diamètre moyen compris entre 0,1 et 1 µm, le parfum étant présent dans la composition en quantités comprises entre 3 et 20% en poids par rapport au poids total de la composition et le rapport en poids entre le(s) lipide(s) des vésicules et le parfum étant compris entre 0,05 et 0,2, le parfum pouvant être mélangé à des adjuvants cosmétiques liposolubles.

Les vésicules sont, de façon connue, délimitées par des couches bimoléculaires ou multimoléculaires d'un

lipide non-ionique. Dans les brevets français 2 315 991 et 2 543 018 sont décrites des dispersions de vésicules de lipides dans une phase aqueuse. Dans les brevets français 2 485 921 et 2 490 504,

on a décrit que les vésicules de lipides non-ioniques stabilisent des dispersions,en phase aqueuse,de liquides non-miscibles à l'eau, en particulier d'huile, sans qu'il soit nécessaire d'ajouter un agent émulsifiant. La présente demande est donc l'application de cette propriété desdites vésicules à la fabrication de compositions parfumantes, dans lesquelles les gouttelettes de parfum sont maintenues sous forme de dispersion à l'aide des vésicules.

Selon l'invention, on a trouvé que les vésicules de lipides non-ioniques ne dénaturent pas la note parfumée du parfum utilisé et que la présence de ces lipides non-ioniques, biomimétiques par rapport à ceux de la peau, apporte une meilleure cohésion à la peau, une grande douceur et un agrément à l'application. On peut donc utiliser la composante parfumée selon l'invention sur une large étendue de peau sans que cela présente d'inconvénient. Les compositions selon l'invention sont stables au stockage à température ambiante ; elles se présentent sous forme de lait ou crème fluide blancs, légèrement brillants pouvant facilement s'étaler sur la peau et de contact agréable.

On a également constaté que la mise en oeuvre des compositions selon l'invention permettait d'améliorer la rémanence du parfum sur la peau et étalait donc dans le temps l'action parfumante de la composition.

La composition parfumée peut contenir jusqu'à 20 % en poids de parfum. Pour des teneurs en perfum supérieures à 20 %, il devient difficile d'obtenir des compositions parfumées stables. Les compositions selon l'invention contiennent, de préférence, de 5 à 16 % en poids de parfum.

Le(s) lipide(s) non-ionique(s) des vésicules représente(nt), de préférence, de 0,5 à 2 % en poids.

Dans les vésicules, les stabilisants anioniques leur donnent une charge négative et améliorent ainsi leur stabilité. Les stabilisants anioniques sont constitués par des esters phosphoriques d'alcools gras en $C_{12}$-$C_{22}$ parmi lesquels on peut citer le phosphate dedicétyle ou de dimyristyle, par des lipoaminoacides comme décrit dans le brevet français 2 597 345 qui sont choisis de préférence parmi ceux dans lesquels le reste acyle $R_5CO$ comporte une chaîne hydrocarbonée $R_5$ en $C_{13}$-$C_{19}$ tels que l'acide palmitoylcollagénique,, l'acide dipalmitoyl-O,N-hydroxyprolinique et le linoléate d'hydroxyproline ainsi que par le sulfate et le phosphate de cholestérol.

Les huiles essentielles utilisées sont des produits obtenus à partir de matières premières d'origine naturelle soit par entraînement à la vapeur d'eau sèche ou humide, soit par des procédés mécaniques, soit par distillation à sec, soit par extraction au moyen de solvants volatils (voir la norme NF-T-75006).
On peut, par exemple, citer l'essence de géranium, les huiles de bergamote, de cèdre, de lavande, de patchouly, de vétiver. Parmi les produits de synthèse parfumantes, on peut citer la vanilline, le linalol, l'alcool phényléthylique et l'acétate de linalyle.

Selon l'invention, le parfum peut contenir des adjuvants liposolubles, tels que des filtres solaires liposolubles par exemple le paradiméthylaminobenzoate de 2-éthyl-hexyle, des substances destinées à améliorer l'état des peaux sèches ou séniles, en particulier des insaponifiables tels que des insaponifiables de soja, d'avocat des vitamines E, F, des agents anti-oxydants ou des huiles animales, végétales, minérales ou synthétiques.

Selon l'invention, la phase aqueuse peut contenir des adjuvants cosmétiques hydrosolubles tels qu'un colorant hydrosolubles, un opacifiant, un filtre solaire hydrosoluble ou un humectant tel que la glycérine, le sorbitol, le pentaérythritol, l'inositol, l'acide pyrrolidone carboxylique et ses sels, ou un composé ayant une activité biologique au niveau de la peau tel que des vitamines ou des extraits animaux ou végétaux.

Des agents gélifiants peuvent éventuellement être introduits à une concentration variant entre 0,1 et 2 % en poids par rapport au poids total de la composition. En présence du gélifiant, on obtient une crème fluide brillante facile à étaler sur la peau et de contact agréable. Parmi les gélifiants utilisables, on peut citer les dérivés de cellulose, tels que l'hydroxyéthylcellulose ; des dérivés d'algues, tels que le satiagum ; des gommes naturelles, telles que l'adragante ; ou encore un mélange d'acides carboxyvinyliques tel que celui commercialisé par GOODRICH BF sous la dénomination commerciale "CARBOPOL". On peut également ajouter à la phase aqueuse un agent conservateur, tel que le parahydroxybenzoate de méthyle, ou un agent régulateur de pH, tel que la triéthanolamine.

La composition parfumante selon l'invention peut être préparée de la façon suivante :

Dans une première étape, on prépare une dispersion de vésicules à partir de lipide(s) non-ionique(s) contenant des stabilisants anioniques et éventuellement du cholestérol dans une phase aqueuse constituée par de l'eau déminéralisée contenant éventuellement un humectant, un composé hydrosoluble ayant une activité biologique au niveau de la peau tel que des vitamines, des extraits animaux ou végétaux ou un agent conservateur, en mettant en oeuvre un procédé conventionnel tel que celui décrit dans les brevets français 2 315 991, 2 543 018 ou 2 221 222.

Dans une seconde étape, une fois la dispersion de vésicules réalisée, on disperse le parfum dans la phase aqueuse continue. La dispersion se fait soit par agitation mécanique, telle qu'avec un ultradisperseur ou un homogénéiseur, soit par passage aux ultrasons.

Les agents cosmétiques liposolubles sont, de préférence, dissous dans le parfum avant son introduction dans la phase aqueuse. Les agents cosmétiques hydrosolubles sont ajoutés à la dispersion de vésicules dans la phase aqueuse en même temps que le parfum ou, après dispersion du parfum. Ils sont, de préférence, introduits sous forme de solution aqueuse.

Les agents cosmétiques liposolubles et/ou hydrosolubles sont présents, de préférence, en quantités comprises entre 0,01 et 5% en poids par rapport au poids total de la composition.

Les exemples donnés ci-dessous permettent de mieux comprendre l'invention.

EXEMPLE 1

1ère étape :

On prépare, dans un premier temps, une dispersion de vésicules lipidiques en phase aqueuse en utilisant un lipide A amphiphile non-ionique de formule :

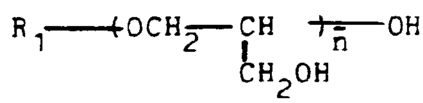

où $R_1$ est un radical cétyle $C_{16}H_{33}$ et $\bar{n}$ = 3.

On réalise la formulation suivante :

| | |
|---|---|
| - Lipide A | 0,475 g |
| - Cholestérol | 0,475 g |
| - Phosphate de dicétyle | 0,05 g |
| - Glycérine | 3 g |
| - Eau | 70 g |

On soumet cette formulation à une agitation mécanique à l'ultradisperseur à 40 000 t/minute pendant 3 minutes.

On obtient,en dispersion aqueuse,des vésicules ayant un diamètre moyen de 0,2µm.

2ème étape :

On introduit dans la dispersion obtenue dans la première étape, 10 g d'huile essentielle de bergamote (exempte de bergaptène) et on soumet à un passage aux ultrasons pendant 2 minutes de façon à obtenir des gouttelettes d'huile essentielle ayant des dimensions comprises entre 0,3 et 0,5µm.

On ajoute ensuite une solution aqueuse contenant :

| | | |
|---|---|---|
| -Parahydroxybenzoate de méthyle............ | 0,2 | g |
| - Eau.................................... | 14,98 | g |
| - Acide polycarboxyvinylique commercialisé sous le nom "CARBOPOL 940" par la société GOODRICH......................... | 0,42 | g |
| - Triéthanolamine........................ | 0,4 | g |

On obtient une composition parfumée ayant l'aspect d'une crème blanche. On a vérifié qu'elle était stable après trois mois de stockage.

EXEMPLE 2

1ère étape :

On prépare des vésicules à partir d'un mélange I constitué par 0,95 g d'un lipide non-ionique B de formule :

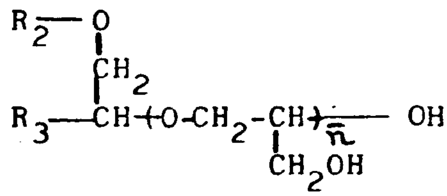

où $R_2$ est un radical lauryle $C_{12}H_{25}$,

$R_3$ est un mélange de radicaux myristyle $C_{14}H_{29}$

et cétyle $C_{16}H_{33}$ et $\bar{n} = 6$ associé à 0,05 g d'un agent anionique (le phosphate de dimyristyle) et mélangé à 8 g d'eau déminéralisée.

On effectue par le procédé décrit dans le brevet français 2 315 991, la dispersion dans un mélange II constitué de 61,63 g d'eau et de 3 g de glycérol.

2ème étape :

On ajoute ensuite le mélange III selon le tableau I, constitué par une huile de bergamote (exempte de bergaptène) éventuellement mélangée à du perhydrosqualène. On soumet la dispersion à un passage aux ultrasons pendant 3 minutes et on ajoute un mélange IV constitué par une solution aqueuse d'acide polycarboxyvinylique vendu par la société GOODRICH BF sous la dénomination commerciale"CARBOPOL®" contenant du parahydroxybenzoate de méthyle et de la triéthanolamine.

Des essais ont été effectués avec des compositions variables données dans le tableau I ci-dessous et on a vérifié la stabilité en jours des crèmes obtenues après deux mois à des températures comprises entre 4 et 45°C.

L'application topique régulière de l'une quelconque des trois crèmes définies par le tableau I confère à la peau de l'utilisateur une grande douceur et une note parfumée agréable.

EXEMPLE 3

On prépare une composition comme dans l'exemple 2 excepté que le mélange IV ne contient pas de"CARBOPOL®". La composition obtenue se présente sous forme d'un lait ayant une excellente stabilité ; l'application topique a les mêmes avantages que pour les crèmes de l'exemple 2.

TABLEAU I

| Mélange | Composition | Essai 1 | Essai 2 | Essai 3 |
|---|---|---|---|---|
| I | Lipide B | 0,95 g | 0,95 g | 0,95 g |
| | Phosphate de dimyristyle | 0,05 g | 0,05 g | 0,05 g |
| | Eau déminéralisée | 8 g | 8 g | 8 g |
| II | Eau déminéralisée | 61,63 g | 61,63 g | 61,63 g |
| | Glycérol | 3 g | 3 g | 3 g |
| III | Huile de bergamote | 5 g | 10 g | 20 g |
| | Perhydrosqualène | 5 g | 0 | 0 |
| IV | Conservateur | 0,2 g | 0,2 g | 0,2 g |
| | Eau déminéralisée | 15,35 g | 15,35 g | 15,35 g |
| | Carbopol ® | 0,42 g | 0,42 g | 0,42 g |
| | Triéthanolamine | 0,4 g | 0,4 g | 0,4 g |
| Stabilité | | Bonne | Bonne | Bonne |

EXEMPLE 4

On définit un parfum L correspondant à la formulation suivante :

| | |
|---|---|
| Huile essentielle de bergamote .................... | 10 g |
| Huile essentielle d'orange ........................ | 6 g |
| Alcool phényléthylique ............................ | 8 g |
| Huile essentielle de bois de rose ................. | 10 g |
| Aldéhyde α-hexyl cinnamique ....................... | 10 g |
| Produit de condensation de :<br>- base de Schiff hydroxycitronellal et<br>- anthralinate de méthyle<br>(produit commercialisé sous le nom de "AURANTIOL ® " vendu par la société GIVAUDAN) ...................... | 8 g |
| Acétate de benzyle ................................ | 5 g |
| Coumarine ......................................... | 10 g |
| Héliotropine ...................................... | 5 g |
| Vanilline ......................................... | 5 g |
| Isométhylionone ................................... | 12 g |
| Mélange de méthylcétones obtenu à partir de l'essence de cèdre traitée (Produit commercialisé sous le nom de "VERTOFIX ® ", vendu par la société "I.F.F.") ........................................ | 11 g |

On ajoute à la dispersion obtenue à la fin de la première étape de t'exemple 2 12 grammes du parfum L que l'on disperse dans le mélange ; on soumet la dispersion aux ultrasons pendant 3 minutes et on ajoute à la dispersion obtenue le mélange IV défini dans la deuxième étape de l'exemple 2 (voir tableau I). On obtient ainsi une crème blanche, dont la stabilité est la même que celle de l'exemple 2.

L'application topique de cette crème donne les mêmes résultats que l'application de la crème de l'exemple 2.

EXEMPLE 5

On ajoute à la dispersion obtenue à la fin de la première étape de l'exemple 2, vingt grammes du parfum L défini à l'exemple 4. On procède ensuite comme indiqué à l'exemple 4. On obtient une crème blanche, qui a sensiblement la même stabilité et les mêmes propriétés d'application que celle de l'exemple 4.

EXEMPLE 6

On définit un parfum O correspondant à la formulation suivante :

| | |
|---|---|
| Huile essentielle de bergamote .................... | 8 g |
| Huile essentielle d'orange ........................ | 8 g |
| Huile essentielle de mandarine .................... | 8 g |
| Huille essentielle de petit grain oranger ....... | 7 g |
| Acétate de linalyle ............................... | 12 g |
| Aldéhyde α-hexylcinnamique ........................ | 11 g |
| Huile essentielle de patchouly .................... | 6 g |
| Musc cétone ....................................... | 6 g |
| Huile essentielle de vétyver ...................... | 11 g |
| Produit "VERTOFIX®" défini à l'exemple 4 .......... | 13 g |
| Isométhylionone ................................... | 10 g |

On ajoute à la dispersion obtenue à la fin de la première étape de l'exemple 2, cinq grammes du parfum O ci-dessus défini et 5 grammes d'eau déminéralisée. On réalise une dispersion et on la soumet aux ultrasons pendant 3 minutes. On ajoute alors à la dispersion un mélange ayant la formulation suivante :

| | |
|---|---|
| commerciale "CARBOPOL®" par la société GOODRICH BF.. | 0,2 g |
| Triéthanolamine ....................................... | 0,2 g |
| Parahydroxybenzoate de méthyle ....................... | 0,2 g |
| Eau déminéralisée ..................................... | 15,77 g |

On obtient ainsi un lait, qui a sensiblement la même stabilité et les mêmes avantages à l'application topique que le lait de l'exemple 3.

## Revendications

1. Composition parfumante à phase continue aqueuse ayant une concentration élevée en parfum, caractérisée par le fait qu'elle contient dans la phase aqueuse continue :

a) des vésicules, qui sont obtenues à partir d'au moins un lipide " non-ionique constitué par un dérivé de polyglycérol, linéaire ou ramifié, de formule :

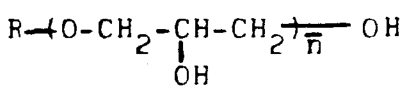

ou

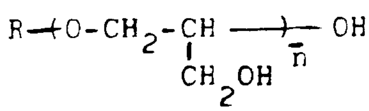

où $\bar{n}$ a une valeur statistique moyenne comprise entre 2 et 6 et où R peut être :

1) ou bien une chaîne aliphatique $R_1$ ou $R_2CO$ dans laquelle $R_1$ est un radical aliphatique, linéaire ou ramifié, en $C_{12}$-$C_{18}$ et dans laquelle $R_2$ est un radical aliphatique, linéaire ou ramifié, en $C_{11}$-$C_{17}$ ;

2) ou bien un radical

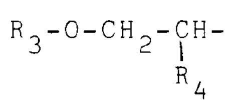 ,

où $R_3$ est un radical $R_1$ ou $R_2CO$ et où $R_4$ est un radical $R_1$, $R_1$ et $R_2$ identiques ou différents, ayant les mêmes significations que ci-dessus indiqué ;

ledit ou lesdits lipide(s) non-ionique(s) étant associé(s) au cholestérol lorsque R a la signification indiquée sous

1) ci-dessus, ledit (ou lesdits) lipide(s) non-ionique(s) étant associé(s) à un agent stabilisant anionique pris dans le groupe formé par les esters phosphoriques d'alcools gras en $C_{12}$-$C_{22}$, les lipoaminoacides, le sulfate et le phosphate de cholestérol, ledit agent stabilisant anionique étant présent à raison de 1 à 10% en poids par rapport au(x) lipide(s), les vésicules ayant un diamètre moyen compris entre 0,01µm et 1 µm et le(s) lipide(s) non-ionique(s), qui les constitue(nt), représentant de 0,2 à 4% en poids de la composition totale ; et

b) un parfum renfermant au moins une huile essentielle naturelle et/ou un produit synthétique à odeur agréable, essentiellement exempt d'alcool en $C_1$-$C_4$, sous forme de gouttelettes ayant un diamètre moyen compris entre 0,1 et 1 µm, le parfum étant présent dans la composition en quantités comprises entre 3 et 20% en poids par rapport au poids total de la composition et le rapport en poids entre le(s) lipide(s) des vésicules et le parfum étant compris entre 0,05 et 0,2 .

2. Composition selon la revendication 1, caractérisée par le fait que la phase aqueuse contient des adjuvants cosmétigues hydrosolubles.

3. Composition selon la revendication 1, caractérisée par le fait que le parfum est mélangé à des adjuvants cosmétiques liposolubles.

4. Composition selon les revendications 1 à 3, caractérisée par le fait que la composition contient de 5 à 16% de parfum.

5. Composition selon l'une des revendications 1 à 4, caractérisée par le fait que le(s) lipide(s) non-ionique(s) des vésicules représente(nt) de 0,5 à 2% en poids de la composition totale.

6. Composition selon l'une des revendications 1 à 5, caractérisée par le fait que l'ester phosphorique d'alcool gras est un phosphate de dicétyle ou de dimyristyle.

7. Composition selon l'une des revendications 1 à 6, caractérisée par le fait que le lipoaminoacide est un lipoaminoacide dans lequel le reste acyle $R_5$-CO- comporte une chaîne hydrocarbonée $R_5$en $C_{13}$ -$C_{19}$ tel que l'acide palmitoyl collagénique, l'acide dipalmitoyl-O,N-hydroxyprolinique ou le linoléate d'hydroxyproline.

8. Composition selon l'une des revendications 1 à 7, caractérisée par le fait que les adjuvants cosmétiques liposolubles ajoutés au parfum sont pris dans le groupe formé par des filtres solaires, des substances destinées à améliorer l'état des peaux sèches ou séniles, des agents anti-oxydants ou des huiles animales, végétales, minérales ou synthétiques.

9. Composition selon l'une des revendications 1 à 8, caractérisée par le fait que les adjuvants cosmétiques hydrosolubles sont tris dans le groupe formé par un colorant hydrosoluble, un opacifiant, un filtre solaire hydrosoluble, un humectant ou un composé ayant une activité biologique au niveau de la peau.

10. Composition selon l'une des revendications 1 à 9, caractérisée par le fait qu'elle contient dans la phase aqueuse un gélifiant.

11. Composition selon la revendication 10, caractérisée par le fait que le gélifiant est pris dans le groupe constitué par les dérivés de cellulose, les dérivés d'algues, les gommes naturelles ou un mélange d'acides polycarboxyvinyliques

## Patentansprüche

1. Riechstoffzusammensetzung mit einer kontinuierlichen wäßrigen Phase und einer hohen Riechstoffkonzentration, **dadurch gekennzeichnet** , daß sie in der wäßrigen Phase enthält:

a) Vesikel, die man aus mindestens einem nicht-ionischen Lipid erhält, welches aus einem geraden oder verzweigten Polyglycerin-Derivat der Formel:

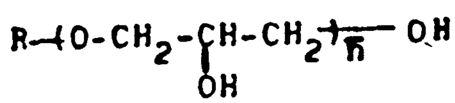

oder

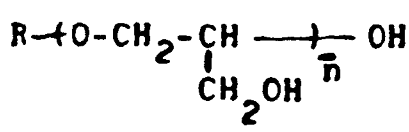

besteht, worin n für einen statistischen Mittelwert zwischen 2 und 6 steht, und R für folgendes stehen kann:

1) eine aliphatische Kette $R_1$ oder $R_2CO$, worin $R_1$ für einen geraden oder verzweigten aliphatischen Rest mit 12 - 18 Kohlenstoffatomen und $R_2$ für einen geraden oder verzweigten aliphatischen Rest mit 11 - 17 Kohlenstoffatomen stehen; oder

2) einen

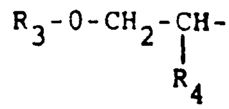

Rest , worin $R_3$ für einen Rest $R_1$ oder $R_2CO$ und $R_4$ for einen Rest $R_1$ stehen, wobei $R_1$ und $R_2$ gleich oder verschieden sind und die oben angegebenen Bedeutungen besitzen;

wobei das (oder die) nicht-ionische(n) Lipid(e) mit Cholesterin assoziiert ist (sind), wenn R die oben unter 1) angegebene Bedeutung besitzt, und wobei das (oder die) nicht-ionische(n) Lipid(e) mit - einem anioniechen Stabilisierungsmittel assoziiert ist (sind), das ausgewählt ist unter Phosphorsäureestern von $C_{12}$-$C_{22}$-Fettalkoholen, Lipoaminosäuren, Cholesterinsulfat und Cholesterinphosphat, und das anionische Stabilisierungsmittel in einer Menge von 1 bis 10 Gew.-%, bezogen auf das (die) Lipid(e), vorhanden ist, wobei die Vesikel einen durchschnittlichen Durchmesser zwischen 0,01 μm und 1 μm behaben und das (die) nicht-ionische(n) Lipid(e), aus welchen die Vesikel bestehen, 0,2 bis 4 Gew.%, bezogen auf das Gesamtgewicht der Zusammensetzung, ausmachen; und

b) ein Parfum, welches aus mindestens einem natürlichen essentiellen Öl und/oder einem synthetischen Produkt mit einem angenehmen Geruch besteht, im wesentlichen keinen Alkohol mit 1 bis 4 Kohlenstoffatomen enthält und In Form von Tröpfchen mit einem mittleren Durchmesser von 0,1 bis 1 μm vorliegt, wobei das Parfum in der Zusammensetzung in einer Menge von 3 bis 20 Gew.-%, bezogen auf das Gesamtgewicht der Zusammensetzung, vorhanden ist und das Gewichtsverhältnis zwischen dem (den) Lipid(en) der Vesikel und dem Parfum von 0,05 bis 0,2 beträgt.

2. Zusammensetzung nach Anspruch 1, dadurch gekennzeichnet, daß die wäßrige Phase wasserlösliche kosmetische Adjuvanzien enthält.

3. Zusammensetzung nach Anspruch 1, dadurch gekennzeichnet, daß das Parfum mit fettlöslichen kosmetischen Adjuvanzien vermischt ist.

4. Zusammensetzung nach den Ansprüchen 1 bis 1, dadurch gekennzeichnet, daß sie 5 bis 16 % Parfum enthält.

5. Zusammensetzung nach einem der Ansprüche 1 bis 4, dadurch gekennzeichnet, daß das (die) nicht-ionische (n) Lipid(e) der Vesikel 0,5 bis 2 Gew.-% der Gesamtzusammensetzung ausmachen.

6. Zusammensetzung nach einem der Ansprüche 1 bis 5, dadurch gekennzeichnet, daß der Phosphorsäureester des Fettalkohols ein Dicetylphosphat oder ein Dimyristylphosphat ist.

7. Zusammensetzung nach einem der Ansprüche 1 bis 6, dadurch gekennzeichnet, daß die Lipoaminosäure eine Lipoaminosäure ist, in welcher der Acylrest $R_5$-CO- eine Kohlenwasserstoffkette $R_5$ mit 13-19 Kohlenstoffatomen, wie Kollagenpalmitinsäure, O,N-Hydroxyprolin-dipalmitinsäure oder Hydroxyprolinlinoleat, aufweist.

8. Zusammensetzung nach einem der Ansprüche 1 bis 7, dadurch gekennzeichnet, daß die dem Parfum beigefügten, fettlöslichen kosmetischen Adjuvanzien ausgewählt sind unter Sonnenfiltern, Stoffen zur Verbesserung des Zustandes trockener oder alter Haut, Antioxidationsmitteln oder tierischen, pflanzlichen, mineralischen oder synthetischen Ölen.

9. Zusammensetzung nach einem der Ansprüche 1 bis 8, dadurch gekennzeichnet, daß die wasserlöslichen kosmetischen Adjuvanzien ausgewählt sind unter einem wasserlöslichen Farbstoff, einem opakmachenden Mittel, einem wasserlöslichen Sonnenfilter, einem feuchthaltenden Mittel oder einem Mittel mit einer biologischen Aktivität gegenüber der Haut.

10. Zusammensetzung nach einem der Ansprüche 1 bis 9, dadurch gekennzeichnet, daß sie ein gelbildendes Mittel in der wäßrigen.Phase enthält.

11. Zusammensetzung nach Anspruch 10, dadurch gekennzeichnet, daß daß gelbildende Mittel ausgewählt ist unter Zellulosederivaten, Algiderivaten, natürlichen Gummis oder einem Vinylpolycarbonsäuren-Gemisch.

## Claims

1. A perfume composition, with a continuous aqueous phase, having a high concentration of perfume, characterized in that it contains, in the continuous aqueous phase:

a) vesicles which are obtained from at least one non-ionic lipid consisting of a linear or branched polyglycerol derivative of the formula

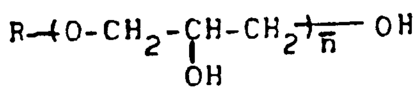

or

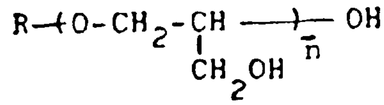

in which $\bar{n}$ has an average statistical value of between 2 and 6 and in which R can be:

1) either an aliphatic chain $R_1$ or $R_2CO$ in which $R_1$ is a linear or branched $C_{12}$-$C_{18}$ aliphatic radical and in which $R_2$ is a linear or branched $C_{11}$-$C_{17}$ aliphatic radical,

2) or a radical

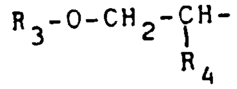

,

in which $R_3$ is a radical $R_1$ or $R_2CO$ and in which $R_4$ is a radical $R_1$, $R_1$ and $R_2$ being identical or different, being defined in the same way as indicated above;

the said non-ionic lipid or lipids being associated with an anionic stabilizer taken from the group comprising phosphoric esters of $C_{12}$-$C_{22}$ fatty alcohols, lipoamino acids and cholesterol sulphate and phosphate, the said anionic stabilizer being present in a proportion of 1 to 10% by weight based on the lipid or lipids, the vesicles having an average diameter of between 0.01 µm and 1 µm and the non-ionic lipid or lipids which form them representing from 0.2 to 4% by weight of the total composition; and

b) a perfume containing at least one pleasant smelling natural essential oil and/or synthetic product, essentially free from $C_1$-$C_4$ alcohol, in the form of droplets having an average diameter of between 0.1 and 1 µm the perfume being present in the composition in amounts of between 3 and 20% by weight based on the total weight of the composition, and the weight ratio of the lipid or lipids of the vesicles to the perfume being between 0.05 and 0.2.

2. A composition according to claim 1, characterized in that the aqueous phase contains water-soluble cosmetic adjuvants.

3. A composition according to claim 1, characterized in that the perfume is mixed with liposoluble cosmetic adjuvants.

4. A composition according to claims 1 to 3, characterized in that the composition contains from 5 to 16% of perfume.

5. A composition according to any one of claims 1 to 4, characterized in that the non-ionic lipid or lipids of the vesicles represent from 0.5 to 2% by weight of the total composition.

6. A composition according to any one of claims 1 to 5, characterized in that the phosphoric ester of a fatty alcohol is a dicetyl or dimyristyl phosphate.

7. A composition according to any one of claims 1 to 6, characterized in that the lipoaminoacid is a lipoaminoacid in which the remaining acyl $R_5$-CO- contains a $C_{13}$-$C_{19}$ hydrocarbon chain $R_5$ such as palmitoylcollagenic acid, dipalmitoyl-O, N-hydroxylprolinic acid or hydroxylproline linoleate.

8. A composition according to any one of claims 1 to 7, characterized in that the liposoluble cosmetic adjuvants added to the perfume are taken from the group consisting of sun filters, substances for improving the condition of dry or ageing skin, antioxidants and animal, vegetable, mineral or synthetic oils.

9. A composition according to any one of claims 1 to 8, characterized in that the water-soluble cosmetic adjuvants are taken from the group consisting of a water-soluble dye, an opacifier, a water-soluble sun filter, a moisturizer and a compound which is biologically active on the skin.

10. A composition according to any one of claims 1 to 9, characterized in that it contains a gelling agent in the aqueous phase.

11. A composition according to claim 10, characterized in that the gelling agent is taken from the group consisting of cellulose derivatives, derivatives of algae, natural gums or a mixture of polycarboxyvinylic acids.